# EUROPEAN PATENT APPLICATION

(11) **EP 4 385 547 A1**
(43) Date of publication of application: **19.06.2024**
(21) Application number: 22214321.6
(22) Date of filing: 16.12.2022
(51) Int. Cl.: A61M 16/00, A61B 5/00

(54) **DETECTING, IDENTIFYING AND ALLEVIATING CAUSES FOR PRESSURE BURDEN USING POSITIVE AIRWAY PRESSURE DEVICES**

(71) Applicant: Koninklijke Philips N.V., 5656 AG Eindhoven (NL)
(72) Inventor: GRASSI, Angela, Eindhoven (NL); KAHLERT, Joachim Johannes, Eindhoven (NL); DOS SANTOS DA FONSECA, Pedro Miguel Ferreira, 5656AG Eindhoven (NL); HENDRIKS, Cornelis Petrus, Eindhoven (NL); VAN ZANTEN, Joyce, Eindhoven (NL)
(74) Representative: Philips Intellectual Property & Standards

(57) **Abstract**

The invention provides a computer implemented method of controlling a positive airway pressure device (109) for providing ventilation support therapy, wherein the positive airway pressure device is configured to deliver pressurized air to a subject, during subject inspiration and expiration, according to a therapeutic pressure protocol. The method comprises receiving physiological data from a physiological sensor arrangement (111); running a detection algorithm to determine an expiration pressure burden based on physiological data; running a pressure adjustment algorithm, wherein the pressure adjustment algorithm is configured to generate a pressure adjustment instruction for adjusting the therapeutic pressure protocol based on the determined expiration pressure burden; and outputting the pressure adjustment instruction to adjust the therapeutic pressure protocol based on the determined expiration pressure burden. In another aspect, the invention provides a ventilation support system (10).

## Description

### FIELD OF THE INVENTION

The present invention relates to positive airway pressure devices for delivering ventilation support therapy and computer implemented methods for controlling positive airway pressure devices. In particular, the invention relates to adapting the pressure settings of a positive airway pressure device.

### BACKGROUND OF THE INVENTION

Positive airway pressure (PAP) devices are systems for providing ventilation support therapy in sleep disordered breathing (SDB) and pulmonary/respiratory diseases.

The inspiration cycle in humans is an active breathing cycle controlled by the breathing muscles (diaphragm and chest muscles). The muscle activity generates a negative pressure in the lungs, which inhale in the air. The expiration cycle for (healthy) persons in rest is a passive outflow of the air. The recoiling of the inflated lungs presses out air from the lungs. During PAP therapy, the recoiling of the chest and the outflow of air is counteracted by the positive pressure of the PAP device. The pressure gradient, which is the driving force to press out the air, is no longer a function of the environmental atmospheric pressure but depends on the pressure provided by the PAP device. The pressure gradient declines with increasing PAP pressure. When the PAP pressure increases, the person tends to experience hyperinflation and has to switch to an active expiration, in which the air is pressed out by the respiration muscle (chest, diaphragm). The positive pressure during expiration is a pressure burden for many people, in particular for high pressure settings, or in patients with a very high sensitivity to expiratory pressure, or in a combination of the two. The need for an active expiration can cause arousals and may make it difficult for patients to fall asleep again, especially during middle-of-the-night awakenings when the homeostatic sleep pressure is relatively low (the patient having already slept for a few hours at the beginning of the night). This represents one of the major causes for a low therapy adherence.

Existing PAP devices differ in the pressure protocol followed.

Continuous positive airway pressure (CPAP) devices provide a constant pressure throughout the treatment period. The pressure is fixed both for inspiratory and expiratory breathing cycles. To alleviate some of the breathing discomfort in the transition from inspiration to expiration, the pressure support in the start of expiration cycle is declined, called C-Flex. The C-flex feature alleviates transitional pressure burden (related to obstructions) but not the burden during the whole expiration cycle and still provides an increased flow at the end of expiration to prevent a collapse of the airway. The A-Flex feature is the same as C-Flex except it increases flow at the end of expiration in a more gradual manner.

Automatic positive airway pressure (autoPAP) devices automatically adapt the pressure settings to rebalance the inspiration tidal volume (i.e., the volume of air inspired in one respiratory cycle) and/or the inhaled minute volume (i.e., the volume of air breathed in (or out) within any 60-second period). That is, the pressure settings are automatically adapted with a view to maintaining a substantially constant inspiration volume over multiple respiratory cycles. The pressure protocol adapts when there is a measured decline in tidal volume (within a predefined pressure range). Typically, autoPAP devices provide the same pressure for inspiration and expiration.

The underlying cause of a decline in tidal volume may be, for example decline of the respiration drive, which may depend on the sleep stage. Another potential underlying cause is an increase of the airway resistance, for instance caused by a narrowing of the airway. A further potential underlying cause is an insufficient work of breathing (WOB), which is impeded by, for example the body position, the person's health condition or by the patient interface.

Bilevel positive airway pressure (BiPAP) devices provide different pressure settings for inspiration and expiration. The pressure protocol can be pre-set for two fixed pressures for the treatment period, or the pressure protocol can be adaptive, similarly to an autoPAP device (in so-called AutoBiPAP devices).

PAP devices are used for various sleep-disordered breathing and respiratory disorders.

For obstructive sleep apnoea (OSA) patients, a positive pressure is essential for pneumatic stenting during inspiration. The target during polysomnography (PSG) titration is to find the appropriate holding pressure, however the pressure implications are not well addressed in expiration.

For central sleep apnoea (CSA) patients the target is to maintain a substantially constant tidal volume. Cardiac and pulmonary implications are not analyzed during a CSA polysomnography.

For chronic obstructive pulmonary disease (COPD) patients, pneumatic stenting is needed during expiration. Positive end expiratory pressure (PEEP) related cardiac implications and oxygen uptake/carbon dioxide release is not well studied.

During a titration sleep study, the physician determines the pressure protocol and prescribes the type of PAP device (CPAP, APAP, BiPAP). In this sleep study, the primary goal is to optimize the airflow in the inspiration cycle. The expiration cycle is not necessarily addressed, and in general is not optimized. In particular, the pressure related burden during expiration is not well considered. However, the pressure burden during expiration can cause hyperinflation, arousals and/or insomnia; it is a major cause for low treatment adherence.

It would be desirable to provide a PAP device that encourages improved treatment adherence.

### SUMMARY OF THE INVENTION

The invention is defined by the claims.

According to examples in accordance with an aspect of the invention, there is provided a computer implemented method of controlling a positive airway pressure device for providing ventilation support therapy, wherein the positive airway pressure device is configured to deliver pressurized air to a subject, during subject inspiration and expiration, according to a therapeutic pressure protocol, the method comprising:
receiving physiological data from a physiological sensor arrangement;
running a detection algorithm to determine an expiration pressure burden based on physiological data;
running a pressure adjustment algorithm, wherein the pressure adjustment algorithm is configured to generate a pressure adjustment instruction for adjusting the therapeutic pressure protocol based on the determined expiration pressure burden; and
outputting the pressure adjustment instruction to adjust the therapeutic pressure protocol based on the determined expiration pressure burden.

The positive airway pressure device may be configured to deliver ventilation support therapy according to a therapeutic pressure protocol. The therapeutic pressure protocol may comprise adapting therapeutic pressure settings to rebalance the tidal volume (i.e., the volume of air inspired or expired in one respiratory cycle) and/or the minute volume (i.e., the volume of air inspired or expired in sixty seconds). By adjusting the therapeutic pressure protocol according to a determined expiration pressure burden, it is possible to improve subject comfort and treatment compliance.

The physiological data may comprise chest movement data and abdominal movement data, the detection algorithm may be configured to process the chest movement data and the abdominal movement data to determine the expiration pressure burden, and the pressure adjustment algorithm may be configured to, in response to determining that the expiration pressure burden is indicative of hyperinflation, generate a pressure adjustment instruction for reducing the air pressure of the pressurized air delivered to a subject during an expiration and/or inspiration cycle.

Reducing the air pressure of the pressurized air delivered to a subject during an inspiration/expiration cycle, in response to determining that the expiration pressure burden indicates hyperinflation, facilitates improved subject comfort.

The physiological data may further comprise body position data and the detection algorithm may be configured to process the chest movement data, the abdominal movement data and the body position data to determine the expiration pressure burden (which may be indicative of hyperinflation).

The physiological data may comprise respiration data and the pressure adjustment algorithm may be configured to, in response to determining that the expiration pressure burden is indicative of active expiration, generate a pressure adjustment instruction for reducing the air pressure of the pressurized air delivered to a subject during an expiration and/or inspiration cycle.

Reducing the air pressure of the pressurized air delivered to a subject during a respiration cycle, in response to determining that the expiration pressure burden indicates active expiration, facilitates improved subject comfort.

The method may further comprise:
receiving validation physiological data for validation the expiration pressure burden;
running a validation algorithm for validating the expiration pressure burden; and
in response to determining that the expiration pressure burden is valid, running the pressure adjustment algorithm.

Validating the determined expiration pressure burden can help to ensure that the air pressure is adjusted appropriately (i.e., when expiration pressure results in excessive subject discomfort). The validation physiological data may comprise heart rate data and/or heart rate variability data.

The detection algorithm may be configured to:
receive heart rate variability data;
process the heart rate variability data to identify an altered venous return flow and, in response to identifying an altered venous return flow, determine a change in heart rate and/or change in heart rate variability; and
in response to determining that the change in heart rate, or the change in heart rate variability, is indicative of a respiration and circulation mismatch, generating a pressure adjustment instruction for reducing the air pressure of the pressurized air delivered to a subject during a respiration cycle.

Reducing the air pressure of the pressurized air delivered to a subject during an expiration cycle, in response to determining that the expiration pressure burden indicates a respiration and circulation mismatch, facilitates improved subject comfort.

Optionally, the pressure adjustment instruction reduces the air pressure of the pressurized air delivered to a subject during multiple respiration cycles.

The detection algorithm may be configured to:
receive, as input, expiration and inspiration flow data;
determine an output, based on the input expiration and inspiration flow data, an expiration pressure burden, wherein the expiration pressure burden is indicative of a leak at an exhaust port at the interface of the positive airway pressure device.

The expiration and inspiration flow data may be determined using an air flow sensor of the PAP device. When subjects change their mask - for instance switching from an oronasal mask to a nasal pillow - the settings of the PAP device are not adapted accordantly. A mismatch between the PAP device settings and the flow characteristics of the mask to be used with the PAP device can cause an expiratory pressure burden. In particular, an expiration pressure burden may arise when amount of air that can be output via the exhaust port (the intentional leak at the exhaust port), typically located at the subject interface (CPAP mask), in insufficient. The leak rate at the exhaust port and the required expiratory respiration drive (expiratory work of breathing) to release the expired air through the exhaust port are correlated.

Running the detection algorithm may further comprise determining a therapeutic pressure recommendation, and the method may further comprise:
analyzing the physiological data to detect initiation of an expiration cycle; and
in response to detecting initiation of an expiration cycle, generating a pressure adjustment instruction for adjusting the therapeutic pressure setting of the positive airway pressure device according to the therapeutic pressure recommendation.

The therapeutic pressure recommendation can be obtained by comparing the physiological data to physiological data obtained during titration. Using this information, it is possible to adjust air pressure in a way that is personalized to the subject.

Analyzing the physiological data to detect initiation of an expiration cycle can facilitate detecting initiation of the expiration cycle in a timely manner.

The method may further comprise:
receiving body position data indicative of a subject's body position;
receiving sleep stage data indicative of a subject's sleep stage;
determining the therapeutic pressure recommendation, based on the body position data and the sleep stage data;
analyzing the physiological data to detect initiation of an expiration cycle; and
in response to detecting initiation of an expiration cycle, generating a pressure adjustment instruction for adjusting the therapeutic pressure setting of the positive airway pressure device according to the therapeutic pressure recommendation.

The therapeutic pressure recommendation can be tailored to the body and sleep stage of the subject. Using this information can help to ensure that air pressure is adjusted in an appropriate manner.

The method may further comprise:
running a pressure tolerance analysis algorithm for analyzing a subject's pressure tolerance during expiration, wherein the pressure tolerance algorithm may be configured to:
receive titration respiration data, wherein the titration respiration data indicates respiration as a function of therapeutic pressure;
receive cortical arousal data; and
determine a therapeutic pressure recommendation, based on the titration respiration data and the cortical arousal data.

Therapeutic pressure recommendation can be determined based on physiological data obtained during titration. Using this information, it is possible to adjust air pressure in a way that is personalized to the subject.

Running the pressure tolerance analysis algorithm may further comprise:
receiving body position data indicative of a subject's body position;
receiving sleep stage data, indicative of a subject's sleep stage; and
for a respective combination of body position and sleep stage, determine a respective maximum tolerated therapeutic pressure.

The therapeutic pressure recommendations generated during titration can be tailored to the body and sleep stage of the subject. Using this information can help to ensure that air pressure is adjusted in an appropriate manner.

The method may further comprise running a titration monitoring algorithm, wherein the titration monitoring algorithm may be configured to:
receive subject data, the subject data comprising data corresponding to at least one parameter indicative of a change in expiration pressure intolerance; and
determine, based on the subject data, a change in expiration pressure intolerance; and
output a re-titration instruction.

In this way, it is possible to ensure therapeutic pressure recommendations and up-to-date and provide appropriate air pressure adjustment.

The method may further comprise running a prediction algorithm for predicting an expiration intolerance event wherein the prediction algorithm may be configured to:
receive, as an input variable, physiological data;
determine, as an output, based on the input variable, a predicted expiration intolerance event; and
the method may further comprise determining, based on the predicted expiration intolerance event, a therapeutic pressure recommendation.

Predicting an expiration intolerance event and generating a therapeutic pressure recommendation based on the prediction, can help to ensure subject comfort.

According to another aspect of the invention, there is provided a processor for use in a ventilation support system, wherein the processor is configured to carry out the steps of the above-described method.

According to another aspect of the invention, there is provided a ventilation support system comprising:
a positive airway pressure device configured to deliver pressurized air to a subject, during subject inspiration and expiration, according to a therapeutic pressure protocol;
a physiological sensor arrangement for obtaining physiological data; and
the above-described processor.

The ventilation support system is configured to determine a pressure burden during expiration and to adjust the pressure setting of the positive airway pressure device according to the detected pressure burden. In this way, the ventilation support system can facilitate improved user comfort, and thereby encourage improved treatment compliance.

The positive airway pressure device may be configured to deliver ventilation support therapy according to a therapeutic pressure protocol. The therapeutic pressure protocol may comprise adapting therapeutic pressure settings to rebalance the tidal volume (i.e., the volume of air inspired or expired in one respiratory cycle) and/or the minute volume (i.e., the volume of air inspired or expired in sixty seconds).

The physiological sensor arrangement may comprise:
a first sensor array for sensing chest movement and abdominal movement, and optionally wherein the physiological sensor arrangement further comprises a second sensor array for sensing body position and/or changes to body position; or
a first sensor array for sensing a respiratory parameter.

The ventilation support system may further comprise a database storing a plurality of subject therapeutic pressure recommendations.

According to another aspect of the invention, there is provided a computer program product comprising computer program code means which, when executed on a computing device having a processing system, cause the processing system to perform all of the steps of the above-described method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a schematic diagram of a ventilation support system;
Fig. 2 shows a flow diagram illustrating a computer implemented method according to one or more embodiments of the invention;
Fig. 3 shows a flow diagram illustrating a computer implemented method according to one or more embodiments of the invention;
Fig. 4 shows a flow diagram illustrating a computer implemented method according to one or more embodiments of the invention;
Fig. 5 shows a flow diagram illustrating a computer implemented method according to one or more embodiments of the invention;
Fig. 6 shows a flow diagram illustrating a computer implemented method according to one or more embodiments of the invention;
Fig. 7 shows a flow diagram illustrating a computer implemented method according to one or more embodiments of the invention;
Fig. 8 shows a flow diagram illustrating a computer implemented method according to one or more embodiments of the invention; and
Fig. 9 shows a flow diagram illustrating a computer implemented method according to one or more embodiments of the invention.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a ventilation support system. The ventilation support system comprises a positive airway pressure device configured to deliver pressurized air to a subject, during subject inspiration and expiration, according to a therapeutic pressure protocol. Further, the system comprises a physiological sensor arrangement for obtaining physiological data and a processor configured to carry out one or more methods described herein of controlling the positive airway pressure device.

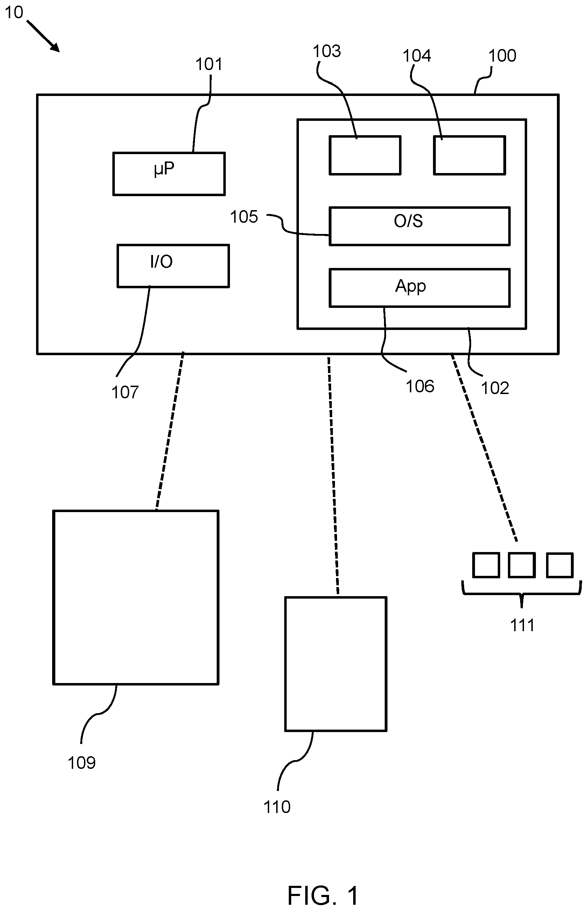

Fig. 1 shows a schematic diagram of a ventilation support system 10 according to an embodiment of the invention. The ventilation support system 10 comprises a positive airway pressure device 109 configured to deliver pressurized air to a subject, during subject inspiration and expiration, according to a therapeutic pressure protocol. The therapeutic pressure protocol determines the air pressure at which pressurized air is to be delivered to a subject during inspiration and/or expiration. In some embodiments, the positive airway pressure device 109 is an auto-PAP device, and the therapeutic pressure protocol automatically adjusts the pressure delivered to a subject during inspiration and/or expiration to maintain a desired tidal volume.

The ventilation support system 10 further comprises a computer 100, comprising a processor configured to carry out any of the methods described herein for controlling the positive airway pressure device 109.

The ventilation support system 10 also comprises a physiological sensor arrangement 111 for obtaining physiological data. The physiological sensor arrangement 111 may comprise sensors for measuring a respiratory parameter (e.g., airflow, duration of inspiration and/or expiration, amount of CO₂, tidal volume). Additionally, or alternatively, the physiological sensor arrangement 111 may comprise sensors for monitoring body movements (e.g., chest movements, abdominal movements, changes to body position during sleep). Further, the physiological sensor arrangement 101 may comprise sensors for measuring parameters indicative of increased pressure-related sympathetic activity (e.g., an electromyography (EMG) sensor, cardiac sensors such as ECG/PPG/BCG/SCG).

Additionally, the ventilation support system 10 comprises a database 110 for storing therapeutic pressure recommendations. The computer 100 is configured to communicate with the database 110 to obtain therapeutic pressure recommendation associated with a user. The therapeutic pressure recommendation may indicate a pressure setting for the positive airway pressure device 109. For example, the therapeutic pressure recommendation may indicate a maximum air pressure to be delivered during inspiration and/or expiration.

The computer 100 may also be configured to communicate with the database 110 to update the therapeutic pressure recommendations stored in the database.

In another aspect, the invention provides a computer implemented method of controlling a positive airway pressure device for providing ventilation support therapy, wherein the positive airway pressure device is configured to deliver pressurized air to a subject, during subject inspiration and expiration, according to a therapeutic pressure protocol. The method comprises receiving physiological data from a physiological sensor arrangement and running a detection algorithm to determine an expiration pressure burden based on physiological data. The method further comprises running a pressure adjustment algorithm, wherein the pressure adjustment algorithm is configured to generate a pressure adjustment instruction for adjusting the therapeutic pressure protocol based on the determined expiration pressure burden and outputting the pressure adjustment instruction to adjust the therapeutic pressure protocol based on the determined expiration pressure burden.

Insufficient leakage of expired air from the exhaust port of a PAP device can give rise to an expiration pressure burden. Insufficient leakage can be detected based on expiration and inspiration flow data. For example, if the expiration data indicates that the amount of air expired is low compared to the amount of air inspired. In an embodiment, the computer receives, as input, physiological data 202 comprising expiration and inspiration flow data. Further, the computer determines 204, as output, based on the input expiration and inspiration flow data, an expiration pressure burden. In this case the expiration pressure burden is indicative of insufficient leakage at an exhaust port at the interface of the positive airway pressure device. In response to determining the presence of insufficient leakage, the computer generates 205 and output 208 a pressure adjustment instruction. The pressure adjustment instruction may adapt the air pressure delivered to the subject during inspiration and/or expiration.

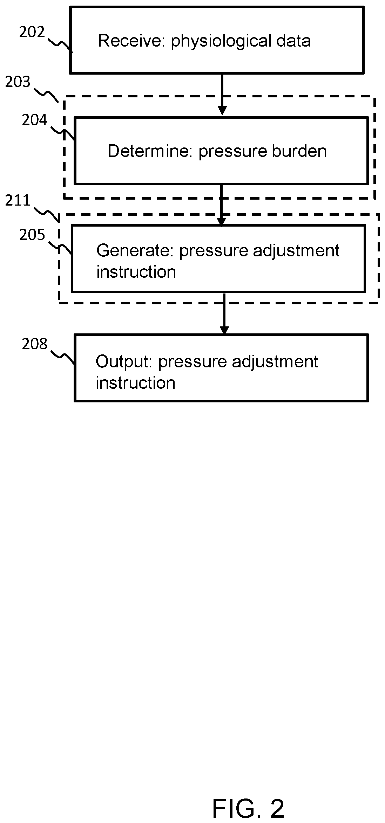

Fig. 2 illustrates a method of controlling a positive airway pressure device according to embodiments of the invention.

The method comprises receiving, 202, physiological data from a physiological sensor arrangement and processing the physiological data to determine an expiration pressure burden 204. The physiological data is processed by running, 203, a detection algorithm configured to determine the expiration pressure burden based on the physiological data.

The expiration pressure burden is a pressure-related burden experienced by the subject when using a positive airway pressure device. For example, the expiration pressure burden may result from hyperinflation of the lungs and/or the need for active expiration. The expiration pressure burden can be detected by monitoring the level of lung inflation (e.g., by measuring abdominal and chest respiratory excursions), airflow and sympathetic activity. The presence of an expiration pressure burden can give rise to increased cortical arousals, impaired sleep and cardiac-related changes. Accordingly, the expiration pressure burden can also be monitored via these indicators.

The detection algorithm 203 analyses the physiological data to determine if the expiration pressure burden is excessive (e.g., the detection algorithm analyses the physiological data to determine if the expiration pressure burden is indicative of user discomfort). In response to determining that the expiration pressure burden is excessive, the computer runs, 211, a pressure adjustment algorithm configured to generate, 205, a pressure adjustment instruction for adjusting the therapeutic pressure protocol of the positive airway pressure device based on the expiration pressure burden determined by the detection algorithm 203. The pressure adjustment instruction is output, 208, by the computer, and communicated to the positive airway pressure device as a control instruction. In this way, the pressure of the air delivered to the user by the positive airway pressure device is automatically adjusted according to the expiration pressure burden experienced by the user.

The pressure adjustment instruction may reduce the air pressure of the pressurized air delivered to the subject during inspiration and/or expiration. This can help to alleviate the pressure burden experienced by the subject. Alternatively, it may be determined that the expiration pressure burden can be alleviated by regulating the subject's breathing pattern (e.g., where it is determined that the subject is breathing too quickly). In that case, the pressure adjustment instruction may increase the air pressure of the pressurized air delivered to the subject during inspiration in order to trigger expiration.

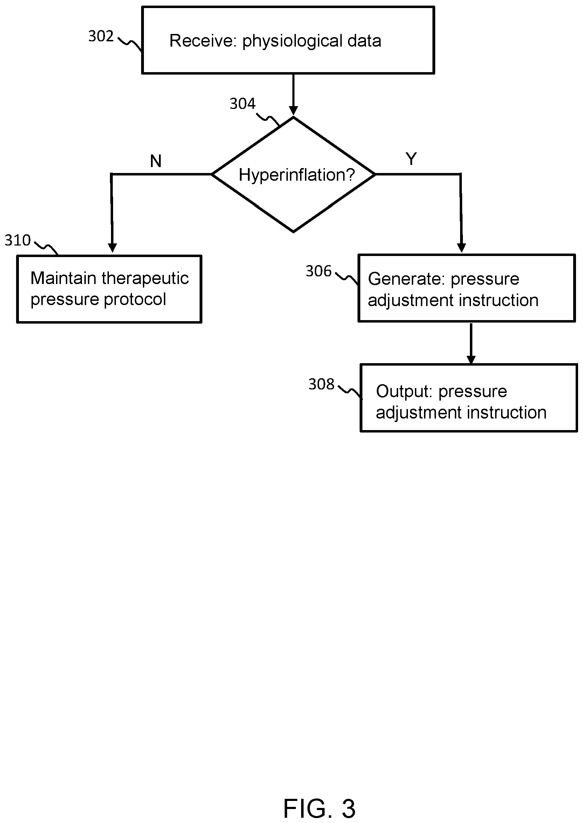

Fig. 3 illustrates an embodiment of a method of controlling a positive airway pressure device. The method comprises receiving, 302, physiological data from a physiological sensor array comprising a physiological sensor for measuring chest movement and a physiological sensor for measuring abdominal movement. The physiological data obtained by the physiological sensor array comprises information indicating the ratio of chest to abdominal breathing.

The expiration pressure burden is determined, 304, by comparing the chest and abdominal movement data and the ratio of chest to abdominal breathing to respective thresholds. When the degree of chest movement, abdominal movement and/or chest to abdominal breathing ratio are above threshold, it is determined that the expiration pressure burden is indicative of hyperinflation. In this case, the computer generates a pressure adjustment instruction for reducing the pressure setting of the positive airway pressure device during inspiration and/or expiration. When the degree of chest movement, abdominal movement and chest to abdominal breathing ratio are below threshold, the physiological data is determined not to be indicative of hyperinflation. Accordingly, no change is made to the existing therapeutic pressure protocol of the positive airway pressure device based on the assessment of the expiration burden.

The chest/abdominal movements can be measured with accelerometers or gyroscopes worn on the user's chest/abdomen. Alternatively, the chest/abdominal excursions can be measured using a camera and/or multiple bed sensors positioned to measure and differentiate between chest and abdominal excursions.

In some embodiments, in response to determining that the expiration pressure burden is indicative of hyperinflation, the expiratory pressure of the positive airway pressure device is reduced in one or several subsequent breath cycles to ease the expiratory outflow of air and to reduce the lung inflation.

The thresholds may be pre-determined user-specific thresholds, established during titration. In some embodiments, the subject's body position and/or body position changes are monitored (e.g., using an accelerometer) to establish the gravitational impact of the chest and abdominal movements. The gravitational impact of the chest and abdominal movements can then be taken into account when determining the expiration pressure burden. That is, the appropriate threshold for determining expiration burden may be selected based on the body position of the subject. For example, for body positions associated with relatively high gravitational impact, the thresholds for chest/abdominal excursions may be lower than for body positions associated with relatively low gravitational impact.

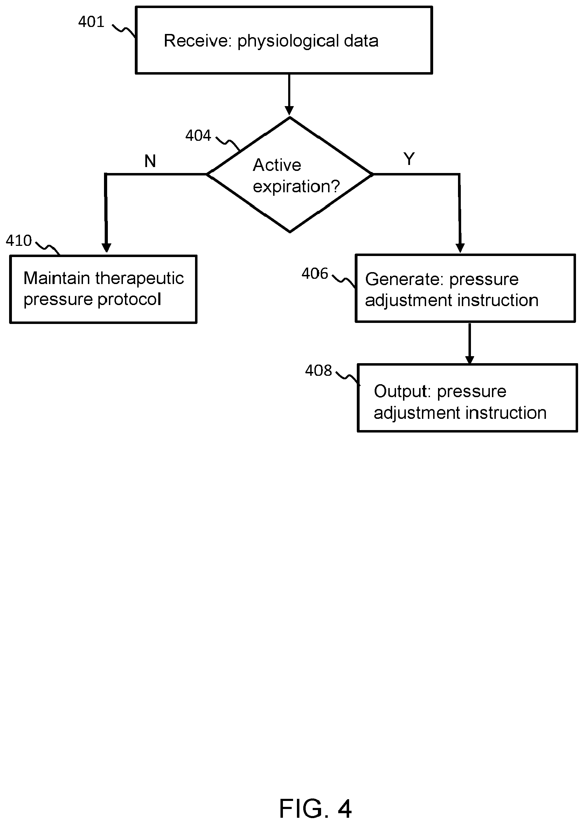

Fig. 4 illustrates an embodiment of a method of controlling a positive airway pressure device. The method comprises receiving, 401, physiological data and determining an expiration pressure burden based on the physiological data. In particular, the method comprises determining, 404, if the physiological data is indicative of active expiration (i.e., muscular driven expiration). The physiological data comprises airflow pattern data received by an airflow sensor arrangement (e.g., an airflow sensor comprised by the positive airway pressure device). The temporal airflow data is analyzed to assess the slope and rise time associated with the expiration cycle. If the slope and/or rise time are above respective thresholds, it is determined that the expiration pressure burden is indicative of active expiration. The thresholds may be pre-determined user-specific thresholds, established during titration.

When the airflow pattern data is indicative of active expiration, the computer generates, 406, a pressure adjustment instruction for reducing the pressure delivered to the subject during the inspiration and/or expiration cycle. Further, the computer outputs, 408, a pressure adjustment instruction If the airflow pattern data is not indicative of active expiration, the existing therapeutic pressure protocol is maintained 410.

In some embodiments, the physiological data comprise airway resistance data measured by a Forced Oscillation Technique (FOT) device. If the measured rise-time in the expiration cycle is equal to the time constant determined by the FOT device the subject is determined to be in passive expiration. If the measured rise-time is shorter than the time constant determined by the FOT system the subject is in active expiration.

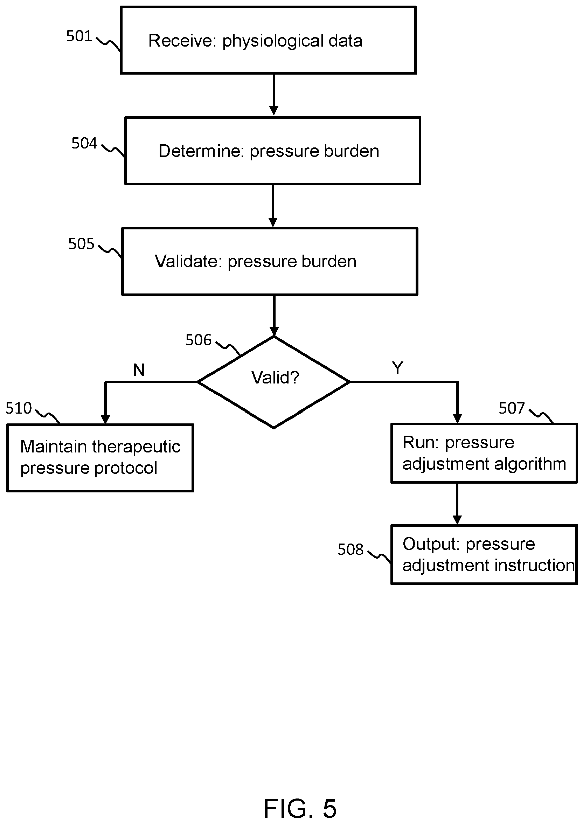

Active expiration is associated with increased sympathetic activity during expiration. In some embodiments, as illustrated in Fig. 5, the method further comprises validating the pressure burden.

The computer receives, 501, physiological data and analyses the physiological data to determine, 504, an expiration pressure burden. The physiological data comprises temporal data relating to a respiratory parameter, such as airflow and/or airway resistance, which can be analyzed to assess whether a subject has transitioned from passive expiration to active expiration. Additionally, the physiological data comprises sympathetic activity data (e.g., EMG data, heart rate data and/or heart rate variability data). This data is used to validate, 506, the finding at step 504.

If it is determined that an expiration burden is present, and the sympathetic activity data correlates with active expiration, the expiration pressure burden is determined to be valid. Accordingly, the computer runs, 507, the pressure adjustment algorithm and outputs, 508, a pressure adjustment instruction.

If it is determined that an expiration burden is present, and the sympathetic activity data does not correlate with active expiration (there is no significant increase in sympathetic activity), the expiration pressure burden is determined to be invalid. In response to determining that the determined expiration is invalid, the computer maintains, 510, the existing therapeutic pressure protocol.

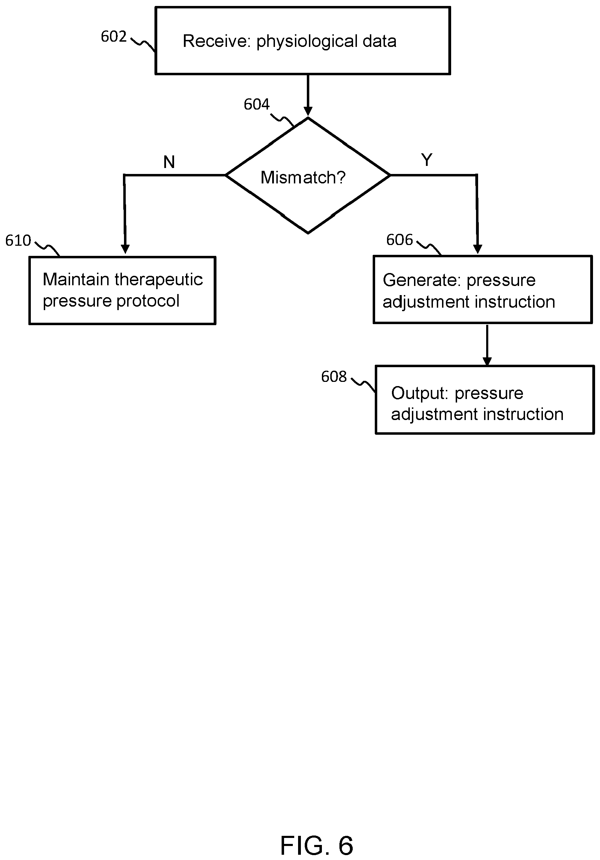

Fig. 6 shows a flow diagram illustrating a method of controlling a positive airway pressure device according to one or more embodiments of the invention. The computer receives, 602, physiological data. The physiological data comprises cardiac data (e.g., heart rate data, cardiac inter-heartbeat data). The cardiac data may be analyzed to determine heart rate variability (HRV) and/or changes in HRV. Changes in heart rate (as determined from HRV) or changes in HRV may be indicative of a mismatch between ventilation and circulation. Accordingly, in the physiological data is analyzed to assess heart rate variability to identify the presence of an expiration pressure burden 604.

For example, HRV is modulated by respiration (along with a number of other factors such as autonomic balance, blood pressure, sleep stages, circadian rhythm etc.). A change in HRV may be indicative that the subject is experiencing a ventilation and circulation mismatch (i.e., an expiration pressure burden). By quantifying the degree of HRV modulation and comparing it to a threshold (which may be specific to the subject) it is possible to assess if the subject is experiencing a ventilation and circulation mismatch 604 (i.e., to identify the presence of an expiration pressure burden). If the degree of modulation is beyond the threshold, a pressure adjustment instruction is generated 606 and output 608 to the positive airway pressure device to reduce the air pressure of the pressurized air delivered to the subject by the positive airway pressure device. If the degree of HRV modulation is not beyond the threshold, the existing therapeutic pressure protocol is maintained 610.

In another example, the degree of HRV itself is assessed (to assess whether changes in heart rate indicative of an expiration burden are present). In particular, HRV is compared to a respective threshold. If HRV is beyond the threshold, a pressure adjustment instruction is generated 606 and output 608 to the positive airway pressure device to reduce the air pressure of the pressurized air delivered to the subject by the positive airway pressure device. If the degree of HRV is not beyond the threshold, the existing therapeutic pressure protocol is maintained 610

As discussed above, the thresholds used to assess the expiration pressure burden may be subject-specific. This information can be obtained by carrying out positive airway pressure titration (or "titration study"), which refers to the process of carrying out testing to determine the level of air pressure needed to resolve the relevant health concern.

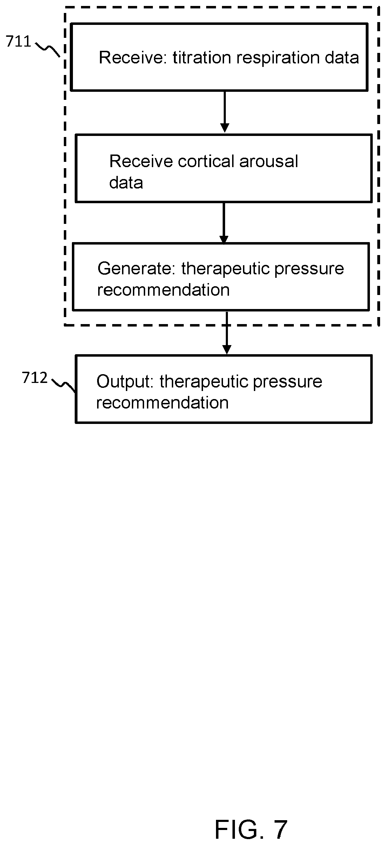

Fig. 7 shows a flow diagram illustrating a method of generating a therapeutic pressure recommendation for use in a method of controlling a positive airway pressure device, using data obtained during a positive airway pressure titration, according to one or more embodiments of the invention.

The titration respiration data comprises data obtained by sensors configured to measure breathing pattern and data obtained by sensors for measuring physiological stress (e.g., heart rate, heart rate variability, SPO2, muscle tone, sympathetic activity, breathing rate, irregular breathing pattern, involuntary body movements, sweating). The data is obtained during a titration session, during which the pressure settings of the positive airway pressure device are ramped up and down according to a titration protocol, and the corresponding changes to physiological stress parameters are recorded. The information obtained during titration is used to determine a pressure response profile for the subject. For example, a biomechanical model may be developed for the subject based on the information obtained during titration. In the biomechanical model, the measured physiological parameters are used to calculate pressure intolerance thresholds and to forecast a sympathetic response. The biomechanical model can therefore be used to provide a reference table for the positive airway pressure device settings.

The method comprises running a therapeutic pressure recommendation algorithm 711 configured to receive titration respiration data and cortical arousal data. This data is analyzed to generate a therapeutic pressure recommendation e.g., the maximum therapeutic pressure that does not trigger arousals due to hyperinflation and/or active expiration. The therapeutic pressure recommendation is then output 712 (e.g., the therapeutic pressure recommendation may be stored in a database, which can be accessed a computer for controlling the positive airway pressure device).

During the titration study, expiratory pressure is varied progressively, at different timepoints during the night. The subject is monitored to detect cortical arousals associated with the variations in expiratory pressure. This data can then be analyzed, using the therapeutic pressure recommendation algorithm, to determine the maximum expiratory pressure that did not trigger arousals (e.g., due to hyperinflation and/or active expiration) over the titration period. During the titration study, if the subject is wearing a nasal mask or nasal pillow expiratory pressure intolerance may be caused by an increased nasal airway resistance. In that case, the subject and/or care giver can be alerted to switch to an oronasal mask.

In another example in which titration study data is used to determine expiration pressure thresholds, the titration study can be conducted by varying the expiratory pressure progressively, at different timepoints during the titration period, to obtain a set of expiration pressure thresholds. The subject's body position and sleep stage are monitored, and expiration pressure is varied for a plurality of body position-sleep stage combinations. The subject is monitored to detect cortical arousals associated with the variations in expiratory pressure, in order to determine an expiration pressure threshold (e.g., a maximum pressure that did not trigger arousals due to hyperinflation and/or active expiration) associated with the body position-sleep stage combination. Accordingly, each expiration pressure threshold in the set corresponds to a particular body position-sleep stage combination.

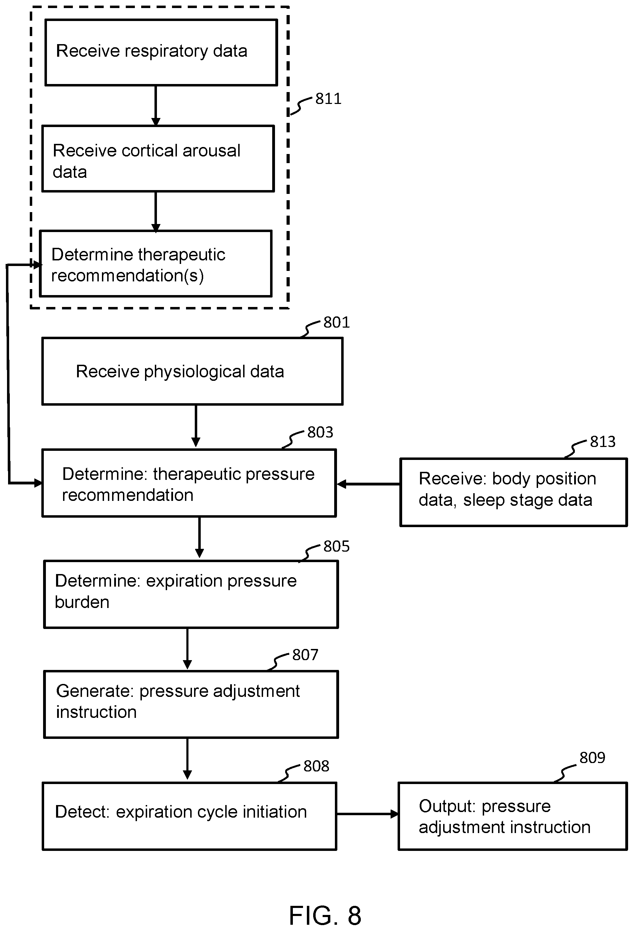

Fig. 8 shows a flow diagram illustrating a method of controlling a positive airway pressure device according to one or more embodiments of the invention.

The method comprises running a therapeutic recommendation algorithm, 811, to determine one or multiple therapeutic recommendations based on respiratory data and cortical arousal data obtained during a titration study. For example, the therapeutic recommendation algorithm 811 determines a set of expiration pressure thresholds, each expiration pressure threshold corresponding to a respective body position-sleep stage combination.

The method further comprises receiving, 813, body position and sleep position data obtained during normal use of the positive airway pressure device (as opposed to during a titration study). Body position can be monitored using sensors (e.g. an accelerometer attached to the subject) and the current sleep stage can be monitored based on respiratory activity (e.g. as measured according to an airflow signal, accelerometer/gyroscope worn around the thorax/abdomen, camera, sensor mounted to the bed) and/or based on cardiac activity (measured with e.g. a PPG/ECG/BCG/SCG sensor).

To determine (or "obtain"), 803, the therapeutic pressure recommendation the current body position and sleep stage data is analyzed, to identify the current body position-sleep stage combination. The therapeutic pressure recommendation corresponding to that body position-sleep stage combination, generated by the therapeutic recommendation algorithm, is identified. If the current body position-sleep stage combination, as determined from the body position and sleep stage data, does not correspond to a body position-sleep stage combination for which a therapeutic pressure recommendation was determined during titration, a default pressure may be used. For example, the lowest pressure amongst all the for therapeutic pressure recommendations from the set may be used.

The physiological data received in step 801 is analyzed to determine, 805, an expiration pressure burden. For example, the expiration pressure burden may be information indicating that the subject is experiencing hyperinflation and/or active expiration.

If it is determined that an expiration pressure burden is present, a pressure adjustment instruction is generated 807.

The computer analyses the physiological data to detect, 808, initiation of an expiration cycle and, upon detection of a switch to expiration, outputs, 809, the pressure adjustment instruction to control the pressure settings of the positive airway pressure device to adjust the therapeutic pressure in line with the therapeutic pressure recommendation (e.g., by decreasing therapeutic pressure to a value below the maximum pressure that did not trigger arousals due to hyperinflation and/or active expiration during titration).

In some embodiments, parameters indicative of a deterioration of sleep quality resulting from increasing expiratory pressure intolerance can are monitored. Since a subject's characteristics change over time, their sensitivity to expiratory pressure may also increase. This may be a temporary change (e.g. during a period of increased stress, etc) or a permanent one (e.g. due to a natural ageing process, or a change in e.g. body mass). By monitoring characteristics indicative of this change using sensors, or allowing the subject to input data describing their experience of expiratory pressure intolerance, it is possible to assess whether it would be appropriate to update the titration data. In response to determining that re-titration is appropriate, trigger a re-titration session.

Accordingly, in some embodiments the method further comprises running a titration monitoring algorithm. The titration monitoring algorithm is configured to receive subject data, the subject data comprising data corresponding to at least one parameter indicative of a change in expiration pressure intolerance. Additionally, the titration monitoring algorithm is configured to determine, based on the subject data, a change in expiration pressure intolerance and output a re-titration instruction.

The parameters indicative of a deterioration in sleep quality can be measured by monitoring arousals (e.g. with an EEG sensor, with a cardiac and/or respiratory surrogate measure of autonomic arousals, and/or using subject inputs).

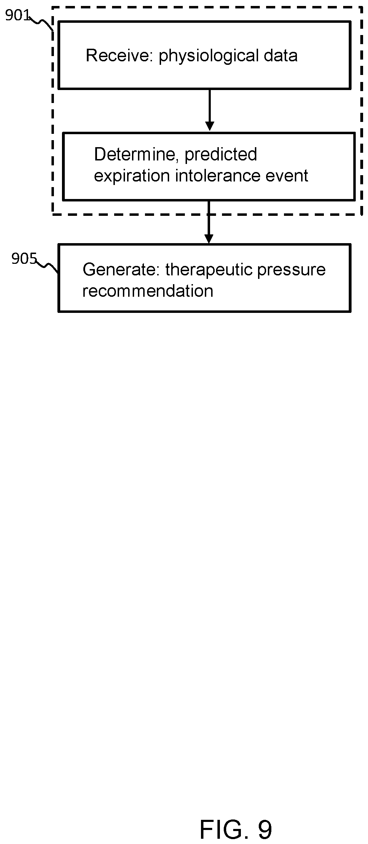

Fig. 9 shows a flow diagram illustrating a method of determining a therapeutic pressure recommendation.

The relationship between physiological signals, arousals and expiratory pressure intolerance response may be complex. In some embodiments, the therapeutic pressure recommendation can be determined using an artificial neural network (ANN). The ANN can be trained to discover relationships between features in the physiological signals, subject characteristics, device parameters (from titration data and with respect to normal usage) and upcoming arousals.

Accordingly, in some embodiments the method may comprise determining a therapeutic pressure recommendation by running a prediction algorithm. The prediction algorithm may be configured to receive, as an input variable, physiological data and determine, as an output, based on the input variable, a predicted expiration intolerance event.

The method may further comprise determining, based on the predicted expiration intolerance event, a therapeutic pressure recommendation. For example, if the predicted expiration intolerance event involves active expiration and/or hyperinflation the therapeutic pressure recommendation may comprise reducing the pressure setting for inspiration and/or expiration.

In some embodiments, training data might be initially limited. The system may therefore be configured to use a look-up table, comprising therapeutic pressure recommendations obtained from a titration study. The ANN can be trained to predict and generate therapeutic pressure recommendations using incoming data from new subjects. Once the ANN reaches a required performance, the trained ANN can be implemented to predict expiration intolerance events/appropriate therapeutic pressure recommendations.

Referring to Fig. 1, an example of a computer 100 is shown, within which one or more parts of an embodiment may be employed. Various operations discussed above may utilize the capabilities of the computer 100. For example, one or more parts of a system for processing an image with a CNN may be incorporated in any element, module, application, and/or component discussed herein. In this regard, it is to be understood that system functional blocks can run on a single computer or may be distributed over several computers and locations (e.g., connected via internet).

The computer 100 includes, but is not limited to, PCs, workstations, laptops, PDAs, palm devices, servers, storages, and the like. Generally, in terms of hardware architecture, the computer 100 may include one or more processors 101, memory 102, and one or more I/O devices 107 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 101 is a hardware device for executing software that can be stored in the memory 102. The processor 101 can be virtually any custom made or commercially available processor, a central processing unit (CPU), a digital signal processor (DSP), or an auxiliary processor among several processors associated with the computer 100, and the processor 101 may be a semiconductor-based microprocessor (in the form of a microchip) or a microprocessor.

The memory 102 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 102 may incorporate electronic, magnetic, optical, and/or other types of storage media. Note that the memory 102 can have a distributed architecture, where various components are situated remote from one another, but can be accessed by the processor 101.

The software in the memory 102 may include one or more separate programs, each of which comprises an ordered listing of executable instructions for implementing logical functions. The software in the memory 102 includes a suitable operating system (O/S) 105, compiler 104, source code 103, and one or more applications 106 in accordance with exemplary embodiments. As illustrated, the application 106 comprises numerous functional components for implementing the features and operations of the exemplary embodiments. The application 106 of the computer 100 may represent various applications, computational units, logic, functional units, processes, operations, virtual entities, and/or modules in accordance with exemplary embodiments, but the application 106 is not meant to be a limitation.

The operating system 105 controls the execution of other computer programs, and provides scheduling, input-output control, file and data management, memory management, and communication control and related services. It is contemplated by the inventors that the application 106 for implementing exemplary embodiments may be applicable on all commercially available operating systems.

Application 106 may be a source program, executable program (object code), script, or any other entity comprising a set of instructions to be performed. When a source program, then the program is usually translated via a compiler (such as the compiler 104), assembler, interpreter, or the like, which may or may not be included within the memory 102, so as to operate properly in connection with the O/S 105. Furthermore, the application 106 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 107 may include input devices such as, for example but not limited to, a mouse, keyboard, scanner, microphone, camera, etc. Furthermore, the I/O devices 107 may also include output devices, for example but not limited to a printer, display, etc. Finally, the I/O devices 107 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 107 also include components for communicating over various networks, such as the Internet or intranet.

If the computer 100 is a PC, workstation, intelligent device or the like, the software in the memory 102 may further include a basic input output system (BIOS) (omitted for simplicity). The BIOS is a set of essential software routines that initialize and test hardware at startup, start the O/S 105, and support the transfer of data among the hardware devices. The BIOS is stored in some type of read-only-memory, such as ROM, PROM, EPROM, EEPROM or the like, so that the BIOS can be executed when the computer 100 is activated.

When the computer 100 is in operation, the processor 101 is configured to execute software stored within the memory 102, to communicate data to and from the memory 102, and to generally control operations of the computer 100 pursuant to the software. The application 106 and the O/S 105 are read, in whole or in part, by the processor 101, perhaps buffered within the processor 101, and then executed.

When the application 106 is implemented in software it should be noted that the application 106 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 106 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

The positive airway pressure device may be an auto-PAP device, a Bi-PAP device or an auto-BIPAP device.

Although Fig. 8 illustrates a method of obtaining a therapeutic pressure recommendation based on physiological data indicative of body position and sleep stage, it will be appreciated with other physiological data can be used, as long as the titration physiological data includes a therapeutic pressure recommendation corresponding to a particular parameter measured in the physiological data.

## Claims

1. A computer implemented method of controlling a positive airway pressure device for providing ventilation support therapy, wherein the positive airway pressure device is configured to deliver pressurized air to a subject, during subject inspiration and expiration, according to a therapeutic pressure protocol, the method comprising:
receiving physiological data from a physiological sensor arrangement;
running a detection algorithm to determine an expiration pressure burden based on physiological data;
running a pressure adjustment algorithm, wherein the pressure adjustment algorithm is configured to generate a pressure adjustment instruction for adjusting the therapeutic pressure protocol based on the determined expiration pressure burden; and
outputting the pressure adjustment instruction to adjust the therapeutic pressure protocol based on the determined expiration pressure burden.

2. The method of claim 1, wherein the physiological data comprises chest movement data and abdominal movement data, the detection algorithm is configured to
process the chest movement data and the abdominal movement data to determine the expiration pressure burden, and the pressure adjustment algorithm is configured to, in response to determining that the expiration pressure burden is indicative of hyperinflation, generate a pressure adjustment instruction for reducing the air pressure of the pressurized air delivered to a subject during an expiration and/or inspiration cycle.

3. The method of any preceding claim, wherein the physiological data comprises respiration data and the pressure adjustment algorithm is configured to, in response to determining that the expiration pressure burden is indicative of active expiration, generate a pressure adjustment instruction for reducing the air pressure of the pressurized air delivered to a subject during an expiration and/or inspiration cycle.

4. The method of claim 3, further comprising:
receiving validation physiological data for validation the expiration pressure burden;
running a validation algorithm for validating the expiration pressure burden; and
in response to determining that the expiration pressure burden is valid, running the pressure adjustment algorithm.

5. The method of any preceding claim, wherein the detection algorithm is configured to:
receive heart rate variability data;
process the heart rate variability data to identify an altered venous return flow and, in response to identifying an altered venous return flow, determining a change in heart rate and/or change in heart rate variability; and
in response to determining that the change in heart rate, or the change in heart rate variability, is indicative of a respiration and circulation mismatch, generating a pressure adjustment instruction for reducing the air pressure of the pressurized air delivered to a subject during a respiration cycle.

6. The method of any preceding claim, wherein the detection algorithm is configured to:
receive, as input, expiration and inspiration flow data;
determine an output, based on the input expiration and inspiration flow data, an expiration pressure burden, wherein the expiration pressure burden is indicative of insufficient leakage at an exhaust port at the interface of the positive airway pressure device.

7. The method of any preceding claim, wherein running the detection algorithm further comprises determining a therapeutic pressure recommendation, and the method further comprises:
analyzing the physiological data to detect initiation of an expiration cycle; and
in response to detecting initiation of an expiration cycle, generating a pressure adjustment instruction for adjusting the therapeutic pressure setting of the positive airway pressure device according to the therapeutic pressure recommendation.

8. The method of claim 7, further comprising:
receiving body position data indicative of a subject's body position;
receiving sleep stage data indicative of a subject's sleep stage;
determining a therapeutic pressure recommendation, based on the body position data and the sleep stage data;
analyzing the physiological data to detect initiation of an expiration cycle; and
in response to detecting initiation of an expiration cycle, generating a pressure adjustment instruction for adjusting the therapeutic pressure setting of the positive airway pressure device according to the therapeutic pressure recommendation.

9. The method of any preceding claim, further comprising:
running a pressure tolerance analysis algorithm for analyzing a subject's pressure tolerance during expiration, wherein the pressure tolerance algorithm is configured to:
receive titration respiration data, wherein the titration respiration data indicates respiration as a function of therapeutic pressure;
receive cortical arousal data; and
determine a therapeutic pressure recommendation, based on the titration respiration data and the cortical arousal data.

10. The method of claim 9, wherein running the pressure tolerance analysis algorithm further comprises:
receiving body position data indicative of a subject's body position;
receiving sleep stage data, indicative of a subject's sleep stage; and
for a respective combination of body position and sleep stage, determine a respective maximum tolerated therapeutic pressure.

11. The method of any preceding claim, further comprising running a titration monitoring algorithm, wherein the titration monitoring algorithm is configured to:
receive subject data, the subject data comprising data corresponding to at least one parameter indicative of a change in expiration pressure intolerance; and
determine, based on the subject data, a change in expiration pressure intolerance; and
output a re-titration instruction.

12. The method of any preceding claim, further comprising running a prediction algorithm for predicting an expiration intolerance event wherein the prediction algorithm is configured to:
receive, as an input variable, physiological data;
determine, as an output, based on the input variable, a predicted expiration intolerance event; and
the method further comprises determining, based on the predicted expiration intolerance event, a therapeutic pressure recommendation.

13. A processor for use in a ventilation support system, wherein the processor is configured to carry out the steps of any of claims 1 to 12.

14. A ventilation support system comprising:
a positive airway pressure device configured to deliver pressurized air to a subject, during subject inspiration and expiration, according to a therapeutic pressure protocol;
a physiological sensor arrangement for obtaining physiological data; and
the processor of claim 13.

15. A computer program product comprising computer program code means which, when executed on a computing device having a processing system, cause the processing system to perform all of the steps of the method according to any of claims 1 to 12.
